# EUROPÄISCHE PATENTSCHRIFT

(11) **EP 0 402 668 B1**
(45) Veröffentlichungstag und Bekanntmachung des Hinweises auf die Patenterteilung: **24.08.1994**
(21) Anmeldenummer: 90109824.4
(22) Anmeldetag: 23.05.1990
(51) Int. Cl.: B01F 9/00

(54) **Mischvorrichtung für Pasten**
Mixing device for pastes
Dispositif de mélange pour pâtes

(30) Priorität: 15.06.1989 DE 8907335 U
(43) Veröffentlichungstag der Anmeldung: 19.12.1990
(73) Patentinhaber: THERA Patent GmbH & Co. KG Gesellschaft für industrielle Schutzrechte, D-82229 Seefeld (DE)
(72) Erfinder: Herold, Wolf Dietrich, Dr., D-8031 Seefeld 2 (DE); Koran, Peter, Dr., D-8120 Weilheim (DE)
(74) Vertreter: Strehl Schübel-Hopf Groening & Partner

(56) Entgegenhaltungen:
- FR-A- 976 266
- FR-A- 2 240 042
- GB-A- 633 258
- US-A- 1 665 065
- US-A- 3 170 648
- US-A- 4 497 581
- PATENT ABSTRACTS OF JAPAN, Band 7, Nr. 92 (M-208)[1237], 16. April 1983;& JP-A-58 15 665 (SHIKISHIMA CHIPTON K.K.) 29-01-1983
- PATENT ABSTRACTS OF JAPAN, Band 8, Nr. 258 (M-340)[1695], 27. November 1984;& JP-A-59 129 656 (SHIKISHIMA CHIPTON K.K.) 26-07-1984
- PATENT ABSTRACTS OF JAPAN, Band 8, Nr. 210 (M-328)[1647], 26. September 1984;& JP-A-59 97 838 (HIROAKI HIDEYOSHI) 05-06-1984

## Beschreibung

Bei der Anmischung einer Paste aus zwei oder mehreren Komponenten, insbesondere zur Herstellung eines Knochenzements aus einer flüssigen und einer pulverförmigen Komponente, kommt es darauf an, daß eine möglichst homogene Mischung erzeugt wird, die wegen der zu erzielenden Festigkeit nach dem Abbinden völlig blasenfrei sein soll. Die beim Mischvorgang durch die Bewegung des Mischgutes eingebrachte Luft muß daher während oder nach dem Mischvorgang entfernt werden. Handelt es sich um einen Knochenzement, der typischerweise eine Abbindezeit im Minutenbereich hat, so steht für die Zubereitung der Mischung und die Entfernung der Luft nur wenig Zeit zur Verfügung.

Bei einer aus DE-A-3 708 442 bekannten Mischvorrichtung sind die miteinander zu mischenden Komponenten in einem gemeinsamen Behälter enthalten und im Lieferzustand durch eine Trennwand voneinander getrennt, die zunächst zerstört wird. Der Behälter wird dann in einen Halter eingesetzt, der zur Erzeugung einer Schüttelbewegung an einem Rotor um eine zur Rotorachse exzentrische Achse drehbar gelagert ist. Durch elastische Mittel ist der Halter derart gegenüber dem Gehäuse der Vorrichtung verspannt, daß er während der Drehbewegung des Rotors seine generelle Ausrichtung beibehält und dadurch in eine kreisende Hin- und Herbewegung versetzt wird. Nach Beendigung des Mischvorgangs wird der Behälter an eine Vakuumpumpe angeschlossen, um das Gemisch zu entlüften. Sodann wird der Behälter mit einer Ausbringvorrichtung verbunden, die mit einem Stößel auf einen in dem Behälter vorhandenen Kolben einwirkt, um das fertige Gemisch durch eine am anderen Behälterende vorgesehene Ausbringöffnung auszudrücken.

Weitere, ähnliche Mischvorrichtungen sind aus DD-A-94 554 und DE-A-2 734 488 bekannt. Bei der letzteren wird der Mischbehälter auf einem Planentenrad montiert, das mit Sonnenrad und Radkranz eines Planentengetriebes kämmt. Durch periodische Änderung der relativen Winkelstellung zwischen Sonnenrad und Radkranz wird eine intermittierende 180°-Hin- und Herdrehung des Behälters um seine Drehachse relativ zum Rotor bewirkt.

Da die fertig gemischte Paste insbesondere dann, wenn es sich um Knochenzement handelt, eine hoch-viskose knetbare Konsistenz aufweist, lassen sich die beim Mischvorgang eingeschlossenen Luftblasen - wenn überhaupt - nur durch ein extrem hohes Vakuum entfernen. Dabei werden aber flüchtige Bestandteile der Paste entzogen, so daß das Mischungsverhältnis des Zements in unvorhersehbarer Weise verändert und dadurch seine chemischen und physikalischen Eigenschaften beeinträchtigt werden.

Aus US-A-1,665,065 ist eine Zentrifugiermaschine mit den im ersten Teil des Anspruchs 1 angegebenen Merkmalen bekannt, bei der mehrere ein zu mischendes und zentrifugierendes Gut enthaltende Trommeln um eine zentrale Achse rotieren. Durch Druck auf ein Pedal läßt sich die Anordnung etwas anheben, so daß am oberen Ende der Trommelachsen vorhandene Kegelräder in Eingriff mit einem Sonnenrad gebracht werden und die Trommeln sich dann zusätzlich um ihre eigenen Achsen drehen.

Eine ähnliche Anordnung mit einem Planetengetriebe ist aus GB-A-633,258 bekannt, bei der die Achsen einzelner Zylinder jeweils für sich über Handhebel derart ein- und auskuppelbar sind, daß daß sie zusätzlich zu einer Gesamtdrehung um eine zentrale Achse jeweils um ihre eigenen Achsen rotieren.

Der Erfindung liegt die Aufgabe zugrunde, eine Mischvorrichtung für Pasten, insbesondere für Knochenzement, anzugeben, die bei einfacher Funktions- und Handhabungsweise die rasche Herstellung eines homogenen und blasenfreien Gemisches ohne Veränderung des Mischungsverhältnisses gewährleistet.

Gemäß der im Anspruch 1 angegebenen Lösung dieser Aufgabe wird dieselbe Vorrichtung, die zum Mischen der Ausgangskomponenten dient, für einen anschließenden Kompressionsvorgang herangezogen, wobei der den Behälter aufnehmende Halter, der während des Mischvorgangs um seine exzentrische Achse derart rotiert, daß das Gemisch einer hin- und hergehenden Schüttelbewegung ausgesetzt ist, durch Umschaltung einer Getriebeeinrichtung freigegeben wird und sich nun auf eine zu dem Rotor ortsfeste Ausrichtung einstellen kann, in der der Behälterinhalt durch die bei der weiteren Drehung des Rotors erzeugten Zentrifugalkräfte verdichtet wird. Dabei wird die fertig gemischte Paste in Richtung des Kolbens verdichtet, so daß sie anschließend von diesem aus dem Behälter ausgebracht werden kann, ohne daß bei der Vorschubbewegung des Kolbens erneut Luft durch die fertige Paste gedrückt wird.

Gemäß Anspruch 2 ist die Umschaltung vom Misch- in den Kompressionsvorgang bei ununterbrochener Rotordrehung möglich, so daß der zweite Vorgang unmittelbar an den ersten anschließen kann und der gesamte Zeitbedarf minimiert wird.

Vorteilhafte Ausgestaltungen des Getriebes sind in den Ansprüchen 3 bis 8 angegeben. Die Ansprüche 3 und 4 beziehen sich dabei auf eine besonders einfache Gestaltung der Kupplung. Die Maßnahme des Anspruchs 5 bewirkt, daß der Mischbehälter bei jeder Umdrehung des Rotors einmal hin und her bewegt wird. Der Antrieb des den Mischbehälter aufnehmenden Halters kann dabei gemäß Anspruch 6 über Riemen oder gemäß Anspruch 7 über Zahnräder erfolgen. Anspruch 8 betrifft eine einfach realisierbare Fernbetätigung der Kupplung.

Die Maßnahme des Anspruchs 9 ist nicht nur dann sinnvoll, wenn größere Mengen der Paste gleichzeitig benötigt werden; sie resultiert gleichzeitig in einer Auswuchtung des Rotors.

Die Weiterbildungen nach den Ansprüchen 10 und 11 führen zu einer Mischvorrichtung, die den Misch- und den Kompressionsvorgang sowie gegebenenfalls einen vorherigen Ausbringvorgang, in dem die flüssige Komponente aus einer anfänglich getrennten Kammer des Behälters in die die pulverförmige Komponente enthaltende Mischkammer überführt wird, vollautomatisch nacheinander mit den auf die Spezifikation der jeweiligen Paste eingestellten Zeitintervallen durchführt.

Ausführungsbeispiele der Erfindung werden nachstehend anhand der Zeichnungen näher erläutert, deren beide Figuren zwei Varianten einer Vorrichtung zum Mischen und anschließenden Komprimieren einer Paste in schematischer Schnittdarstellung zeigen.

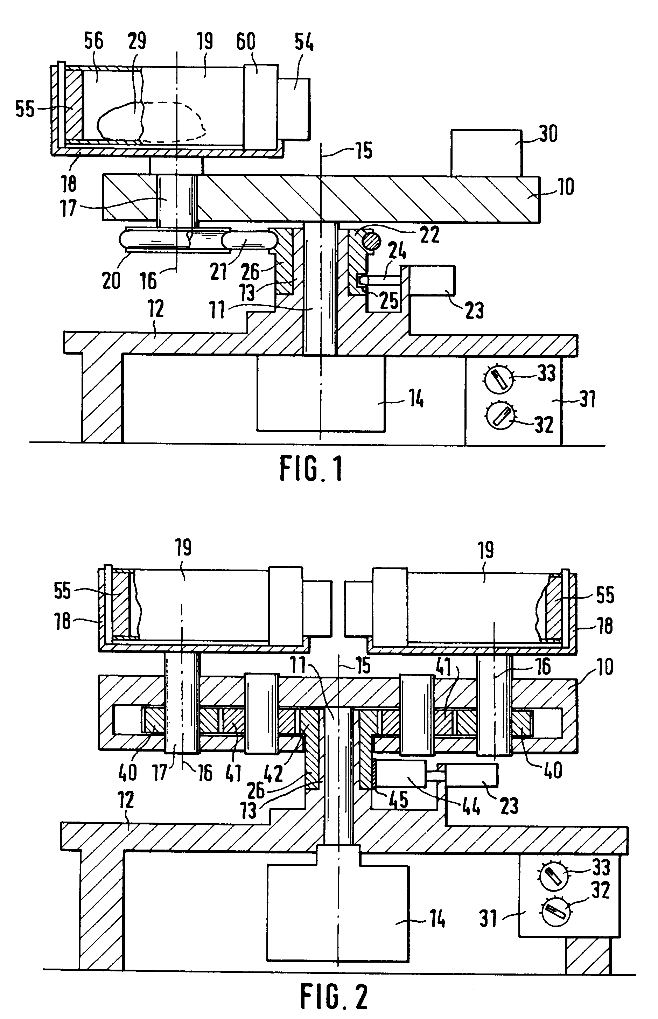

Bei der in Figur 1 gezeigten Vorrichtung ist ein Rotor 10 mit einer Welle 11 verbunden, die in einer mit einem Gehäuse 12 verbundenen Hülse 13 drehbar gelagert ist und von einem in dem Gehäuse 12 untergebrachten Motor 14 angetrieben wird.

In dem Rotor 10 ist um eine zur Achse 15 der Rotorwelle 11 exzentrische und parallele Achse 16 drehbar eine Welle 17 gelagert, an der oberhalb des Rotors ein Halter 18 zur Aufnahme eines Mischbehälters 19 befestigt ist und die unterhalb des Rotors 10 eine Riemenscheibe 20 trägt. Die Riemenscheibe 20 wird über einen Riemen 21 von einer Riemenscheibe 22 angetrieben, die auf der Außenseite der Hülse 13 um die Achse 15 der Rotorwelle 11 drehbar gelagert ist.

An dem Gehäuse 12 ist ferner ein Elektromagnet 23 angebracht, wobei ein mit dem Anker des Elektromagneten 23 verbundener Stößel 24 in eine Vertiefung 25 eingreift, die an der Außenseite eines rohrförmigen Ansatzes 26 der Riemenscheibe 22 vorgesehen ist.

Der Mischbehälter 19 weist eine in Figur 1 rechts gezeigte, beispielsweise die flüssige Komponente eines Knochenzements enthaltende Kammer 54 und links eine die pulverförmige Komponente enthaltende und an ihrem Ende mit einem verschiebbaren Kolben 55 abgeschlossene Mischkammer 56 auf. Die beiden Kammern 54, 56 sind durch eine im Ausgangszustand verriegelte (nicht gezeigte) Ventildichtung voneinander getrennt. Die Dichtung läßt sich durch Verschieben eines an dem Behälter 19 vorgesehenen Rings 60 aus der Verriegelung lösen, so daß die flüssige Komponente dann bei Auftreten einer in Richtung der Mischkammer wirkenden Kraft in diese überführt wird.

Halter 18 und Behälter 19 sind so aneinander angepaßt, daß sich der Behälter 19 nur in einer Ausrichtung in den Halter 18 einsetzen läßt. Ferner sind der Halter 18 und der Behälter 19 derart gestaltet, daß sich der Schwerpunkt der von diesen beiden Bauteilen 18, 19 gebildeten Einheit unabhängig von der jeweiligen Lage des in Figur 1 bei 29 angedeuteten Behälterinhalts außerhalb der Achse 16 auf der Seite des Kolbens 55 befindet.

Zum Ausgleich der von dem Halter 18 mit der Welle 17 und der Riemenscheibe 20 sowie dem gefüllten Mischbehälter 19 gebildeten Unwucht ist ferner an einer zur Achse 16 diametral gegenüber liegenden Stelle des Rotors 10 ein Ausgleichsgewicht 30 befestigt.

Der Motor 14 und der Elektromagnet 23 werden von einer im Gehäuse 12 untergebrachten Steuerschaltung 31 gesteuert, die ein Einstellorgan 32 für das Einschaltintervall des Motors 14 und ein Steuerorgan 33 für den Betätigungszeitpunkt des Elektromagneten 23 aufweist.

Beim Betrieb wird zunächst der Mischbehälter 19 nach Verschieben des Ringes 60 in die die genannte Ventildichtung freigebende Stellung in der vorgeschriebenen Orientierung in den Halter 18 eingesetzt und sodann der Rotor 10 durch Beaufschlagung des Motors 14 in Drehung versetzt. In dieser ersten Phase ist der Stößel 24 des Elektromagneten 23 aus der Vertiefung 25 des Ansatzes 26 zurückgezogen, so daß die Riemenscheibe 22 um die gehäusefeste Hülse 13 und der Halter 18 relativ zu dem Rotor 10 frei drehbar sind. Unabhängig davon, welche Stellung der Halter 18 bei Beginn der Rotation einnimmt, werden die Fliehkräfte des sich drehenden Rotors 10 die aus Halter 18 und Behälter 19 bestehende Einheit in eine Stellung drehen, in der der Kolben 55 von der Rotorachse 15 am weitesten entfernt ist. In dieser in Figur 1 veranschaulichten Stellung wird der Halter 18 mit dem Behälter 19 ohne weitere Eigendrehung bezüglich der Achse 16 mit dem Rotor 10 mitgeführt, so daß die flüssige Komponente aus der Kammer 54 in die Mischkammer 56 überführt wird.

Sodann wird durch Beaufschlagung des Elektromagneten 23 der Stößel 24 in die in Figur 1 gezeigte Stellung in Eingriff mit der Vertiefung 25 in dem Ansatz 26 gebracht, so daß die Riemenscheibe 22 nun stationär gehalten wird. Infolgedessen wird durch die weitere Drehung des Rotors 10 um die Achse 15 der Halter 18 mit dem Mischbehälter 19 relativ zu dem Rotor 10 um die Achse 16 gedreht. Haben die beiden Riemenscheiben 20 und 22 gleichen Durchmesser, wie dies in Figur 1 angenommen ist, so wird der Halter 18 mit dem Mischbehälter 19 bei der Kreisbewegung um die Achse 15 parallel zu sich selbst geführt, so daß das Mischgut einer Abwälzbewegung an den Innenflächen des Mischbehälters 19 unterworfen wird. Dadurch werden die Komponenten innerhalb des Mischbehälters zu einer homogenen Paste miteinander vermischt.

Nach Ablauf des an dem Einstellorgan 33 vorgewählten Zeitintervalls, das mit der ersten Beaufschlagung des Elektromagneten 23 beginnt und sich nach den Eigenschaften der miteinander zu vermischenden Komponenten richtet, beaufschlagt die Steuerschaltung 31 den Elektromagnet 23 erneut so, daß der Stößel 24 zurückgezogen wird. Dadurch wird der Halter 18 wieder frei drehbar und stellt sich auf die in Figur 1 veranschaulichte feste Orientierung relativ zum Rotor 10 ein, so daß der Behälterinhalt 29 nun gegen den Kolben 55 gepreßt wird. Bei diesem Kompressionsvorgang wird die beim Mischen in die Paste gelangte Luft herausgedrückt.

Nach Ablauf des an dem Einstellorgan 32 eingestellten Gesamt-Zeitintervalls hält der Rotor 10 an. Der Mischbehälter 19 wird aus dem Halter 18 herausgenommen und die fertige Paste entnommen.

Die Vorrichtung nach Figur 2 unterscheidet sich von dem Ausführungsbeispiel nach Figur 1 zunächst dadurch, daß anstelle des Ausgleichsgewicht 30 ein zweiter Halter 18 zur Aufnahme eines zweiten Mischbehälters 19 vorgesehen ist. Anstelle der gezeigten zwei können auch mehrere Halter zur Aufnahme von Mischbehältern um die Achse 15 gleichwinklig verteilt an dem Rotor 10 gelagert sein.

Ferner weist das in Figur 2 gezeigte Getriebe, das die Halter 18 während des Mischvorgangs in Eigendrehung relativ zu dem Rotor 10 versetzt, Zahnräder auf, wobei das auf der gehäusefesten Hülse 13 drehbar gelagerte mittlere Zahnrad 42 über jeweils ein Zwischenrad 41 mit einem auf der Welle 17 des betreffenden Halters 18 befestigten Zahnrad 40 in Wirkeingriff steht. Sofern die Zahnräder 40 und 42 gleiche Zähnezahl aufweisen, ergibt sich die gleiche Kinematik, wie sie oben für die Vorrichtung nach Figur 1 beschrieben wurde. Unterscheiden sich die Zähnezahlen, so werden die beiden Halter 18 während der Drehung des Rotors 10 nicht mehr parallel zu sich selbst geführt, sondern führen eine der Rotordrehung überlagerte Drehbewegung aus.

Bei der Vorrichtung nach Figur 2 ist ferner die während des ersten Mischvorgangs das mittlere Zahnrad 42 stationär haltende Kupplung als Bremse ausgestaltet, wobei ein wiederum über einen Elektromagnet 23 ausrückbarer Bremsbacken 44 mit einem Bremsbelag 45 an der Außenfläche des rohrförmigen Ansatzes 26 des mittleren Zahnrades 42 angreift. Diese Gestaltung der Kupplung ist gegenüber der Version nach Figur 1 insofern von Vorteil, als das Wiedereinrücken der Kupplung zur Durchführung eines erneuten Mischvorgangs in jeder beliebigen Stellung des mittleren Zahnrades 42 möglich ist.

Die beiden Halter 18 sind, wie in Figur 2 angedeutet, derart an den über die Zahnräder 40 ... 42 miteinander gekoppelten Wellen 17 befestigt, daß sich bei ausgerückter Kupplung beide Halter 18 in diejenige Stellung drehen können, in der der Kolben 55 in jedem Mischbehälter 29 nach außen weist.

In der obigen Beschreibung ist angenommen, daß der Rotor 18 während der drei Phasen (Überführen der flüssigen Komponente in die Mischkammer 56 - Mischen - Verdichten) stets mit gleicher Drehzahl rotiert. Die Steuerschaltung 31 kann aber auch so ausgelegt sein, daß sie die Motordrehzahl für die einzelnen Phasen variiert, wobei beispielsweise für die Verdichtungsphase eine höhere Drehzahl vorgegeben wird als für den Mischvorgang.

## Patentansprüche

1. Mischvorrichtung, umfassend
einen um eine erste Achse (15) drehbaren Rotor (10) mit einem Halter (18), der um eine zu der ersten Achse (15) exzentrische zweite Achse (16) drehbar an dem Rotor (10) angeordnet ist, und
eine Getriebeeinrichtung (20...22; 40...42), die während der Drehung des Rotors (10) um die erste Achse (15) dem Halter (18) eine Drehbewegung um die zweite Achse (16) erteilt, und die in einen Zustand umschaltbar ist, in dem sie den Halter (18) zur freien Drehung um die zweite Achse (16) freigibt,
dadurch gekennzeichnet,
daß in den Halter (18) ein Komponenten einer Paste enthaltender Behälter (19) einsetzbar ist, der an seinem einen Ende einen Kolben (55) aufweist, und
daß der Schwerpunkt der aus dem Halter (18) und dem in ihn eingesetzten Behälter (19) gebildeten Einheit unabhängig von der Position des Behälterinhalts (29) zwischen der zweiten Achse (16) und dem Kolben (55) liegt.

2. Vorrichtung nach Anspruch 1, dadurch gekennzeichnet, daß die Getriebeeinrichtung (20...22; 40...42) während der Rotordrehung umschaltbar ist.

3. Vorrichtung nach Anspruch 1 oder 2, dadurch gekennzeichnet, daß die Getriebeeinrichtung (20...22; 40...42) ein um die erste Achse (15) drehbares, den Halter (18) zur Drehung um die zweite Achse (16) antreibendes mittleres Rad (22; 42) sowie eine Kupplung (23...25; 23, 44, 45) aufweist, die im eingerückten Zustand das mittlere Rad (22; 42) mit einer ihm eine Relativdrehung bezüglich des Rotors (10) erteilenden Einrichtung (12) verbindet.

4. Vorrichtung nach Anspruch 3, dadurch gekennzeichnet, daß die Kupplung (23...25; 23, 44, 45) eine im eingerückten Zustand das mittlere Rad (22; 42) gegenüber einem stationären Teil (12) der Vorrichtung fixierende Bremse aufweist.

5. Vorrichtung nach Anspruch 3 oder 4, dadurch gekennzeichnet, daß das mittlere Rad (22) ein mit dem Halter (18) verbundenes weiteres Rad (20) über einen Riemen (21) antreibt.

6. Vorrichtung nach Anspruch 3 oder 4, dadurch gekennzeichnet, daß das mittlere Rad (42) ein Zahnrad ist, das ein mit dem Halter (18) verbundenes weiteres Zahnrad (40) antreibt.

7. Vorrichtung nach Anspruch 5 oder 6, dadurch gekennzeichnet, daß das mittlere Rad (22; 42) und das weitere Rad (20; 40) gleiche Durchmesser haben.

8. Vorrichtung nach einem der Ansprüche 1 bis 7, dadurch gekennzeichnet, daß die Kupplung (23...25; 23, 44, 45) elektromagnetisch betätigbar ist.

9. Vorrichtung nach einem der Ansprüche 1 bis 8, dadurch gekennzeichnet, daß der Rotor (10) zwei oder mehrere gleichwinklig um die erste Achse (15) verteilte Halter (18) trägt.

10. Vorrichtung nach einem der Ansprüche 1 bis 9, gekennzeichnet durch eine Zeitsteuerschaltung (31), die nach Ablauf eines ersten einstellbaren Intervalls der Rotordrehung die Getriebeeinrichtung (20...22; 40...42) umschaltet und den Rotor (10) über ein einstellbares zweites Intervall weiterdreht.

11. Vorrichtung nach Anspruch 10, dadurch gekennzeichnet, daß die Zeitsteuerschaltung (31) bei Beginn des ersten Zeitintervalls die Getriebeeinrichtung (20...22; 40...42) aus einem Anfangszustand, in dem der Halter (18) um die zweite Achse (16) frei drehbar ist, in den Zustand umschaltet, in dem sie dem Halter (18) eine Drehung erteilt.

12. Vorrichtung nach Anspruch 11, dadurch gekennzeichnet, daß die Zeitsteuerschaltung (31) die Rotordrehzahl beim Umschalten der Getriebeinrichtung (20...22; 40...42) ändert.

## Claims

1. A mixing apparatus comprising
a rotor (10) rotatable about a first axis (15) and having a holder (18) which is supported on the rotor (10) so as to be rotatable about a second axis (16) eccentric with respect to the first axis (15), and
transmission means (20...22; 40...42) which causes the holder (18) to rotate about the second axis (16) while the rotor (15) rotates about the first axis (15), and which is adapted to be switched-over to a condition in which it allows the holder (18) to rotate freely about the second axis (16),
characterised in
that a container (19) is adapted to be inserted into the holder (18), the container containing components of a paste and having a piston (15) at one of its ends, and
that the centre of gravity of the unit formed by the holder (18) and the container (19) inserted therein is situated between the second axis (16) and the piston (55) irrespective of the position of the container content (29).

2. The apparatus of claim 1, characterised in that the transmission means (20...22; 40...42) is adapted to be switched-over during the rotation of the rotor.

3. The apparatus of claim 1 or 2, characterised in that the transmission means (20...22; 40...42) includes a central wheel (22; 42) which is rotatable about the first axis (15) and drives the holder (18) to rotate about the second axis (16), and a clutch (23...25; 23, 44, 45) which, when engaged, couples the central wheel (22; 42) to means (12) which cause the central wheel to rotate relatively to the rotor (10).

4. The apparatus of claim 3, characterised in that the clutch (23...25; 23, 44, 45) includes a brake which, when engaged, fixes the central wheel (22; 42) with respect to a stationary part (12) of the apparatus.

5. The apparatus of claim 3 or 4, characterised in that the central wheel (22) drives via a belt (21) a further wheel (20) connected to the holder (18).

6. The apparatus of claim 3 or 4, characterised in that the central wheel (42) is a gear which drives a further gear (40) connected to the holder (18).

7. The apparatus of claim 5 or 6, characterised in that the central wheel (22; 42) and the further wheel (20; 40) have equal diameters.

8. The apparatus of any of claims 1 to 7, characterised in that the clutch (23...25; 23, 44, 45) is electro-magnetically actuable.

9. The apparatus of any of claims 1 to 8, characterised in that the rotor (10) carries two or more holders (18) equi-angularly spaced about the first axis (15).

10. The apparatus of any of claims 1 to 9, characterised by a timing circuit (31) for switching-over the transmission means (20...22; 40...42) upon expiry of a first adjustable time interval and causing the rotor (10) to continue rotating during a second adjustable interval.

11. The apparatus of claim 10, characterised in that the timing circuit (31), at the beginning of the first interval, switches-over the transmission means (20...22; 40...42) from an initial condition, in which the holder (16) is freely rotatable about the second axis (16) to the condition in which it causes the holder (16) to rotate.

12. The apparatus of claim 11, characterised in that the timing circuit (31) changes the rotor speed when switching-over the transmission means (20...22; 40...42).

## Revendications

1. Dispositif de mélange, comprenant un rotor (10) tournant autour d'un premier axe (15) et muni d'un support (18) monté sur le rotor (10) de manière à pouvoir tourner autour d'un second axe (16) excentré par rapport au premier axe (15), et un mécanisme de transmission (20 à 22; 40 à 42) qui, pendant la rotation du rotor (10) autour du premier axe (15), imprime au support (18) un mouvement de rotation autour du second axe (16) et peut être commuté dans un état dans lequel il libère le support (18) pour que celui-ci puisse tourner librement autour du second axe (16), caractérisé en ce que dans le support (18) peut être inséré un récipient (19) qui contient une pâte et comporte à son extrémité un piston (55), et que le centre de gravité de l'unité composée du support (18) et du récipient (19) inséré dans ce dernier se situe, indépendamment de la position du contenu (29) du récipient, entre le second axe (16) et le piston (55).

2. Dispositif selon la revendication 1, caractérisé en ce que le mécanisme de transmission ((20 à 22; 40 à 42) peut être commuté pendant la rotation du rotor.

3. Dispositif selon l'une des revendications 1 ou 2, caractérisé en ce que le mécanisme de transmission (20 à 22; 40 à 42) comprend une roue (22; 42) qui peut tourner autour du premier axe (15) et entraîne le support (18) en rotation autour du second axe (16), ainsi qu'un embrayage (23 à 25; 23, 44, 45) qui, à l'état embrayé, assure l'accouplement de la roue médiane (22; 42) avec un dispositif (12) qui lui confère une rotation relative par rapport au rotor (10).

4. Dispositif selon la revendication 3, caractérisé en ce que l'embrayage (23 à 25; 23, 44, 45) comprend un frein qui, à l'état embrayé, bloque la roue médiane (22; 42) par rapport à un élément stationnaire (12) du dispositif.

5. Dispositif selon l'une des revendications 3 ou 4, caractérisé en ce que la roue médiane (22) entraîne par l'intermédiaire d'une courroie (21) une autre roue (20) couplée avec le support (18).

6. Dispositif selon l'une des revendications 3 ou 4, caractérisé en ce que la roue médiane (42) est une roue dentée qui entraîne une autre roue dentée (40) couplée avec le support (18).

7. Dispositif selon l'une des revendications 5 ou 6, caractérisé en ce que la roue médiane (22; 42) et l'autre roue (20; 40) ont des diamètres identiques.

8. Dispositif selon l'une des revendications 1 à 7, caractérisé en ce que l'embrayage (23 à 25; 23, 44, 45) peut être commandé de manière électromagnétique.

9. Dispositif selon l'une des revendications 1 à 8, caractérisé en ce que le rotor (10) porte deux ou plusieurs supports (18) répartis à des intervalles angulaires identiques autour du premier axe (15).

10. Dispositif selon l'une des revendications 1 à 9, caractérisé en ce qu'il comprend un circuit de synchronisation (31) qui, au bout d'un premier intervalle réglable de la rotation du rotor, commute le mécanisme de transmission (20 à 22; 40 à 42) et fait avancer le rotor (10) par l'intermédiaire d'un second intervalle réglable.

11. Dispositif selon la revendication 10, caractérisé en ce que le circuit de synchronisation (31) commute le mécanisme de transmission (20 à 22; 40 à 42) au début du premier intervalle de temps, d'un état de départ dans lequel le support (18) peut tourner librement autour du second axe (16), à l'état dans lequel il imprime audit support (18) un mouvement de rotation.

12. Dispositif selon la revendication 11, caractérisé en ce que, lors de la commutation du mécanisme de transmission (20 à 22; 40 à 42), le circuit de synchronisation (31) modifie la vitesse de rotation du rotor.
